# EUROPÄISCHE PATENTSCHRIFT

(11) **EP 2 979 718 B1**
(45) Veröffentlichungstag und Bekanntmachung des Hinweises auf die Patenterteilung: **28.03.2018**
(21) Anmeldenummer: 15168987.4
(22) Anmeldetag: 22.05.2015
(51) Int. Cl.: A61M 15/00

(54) **VORSCHALTKAMMER MIT BEDIENELEMENT FÜR INHALATOR**
SPACER WITH ACTUATION ELEMENT FOR AN INHALER
CHAMBRE D'INHALATION DOTÉE D'ÉLÉMENT DE COMMANDE POUR INHALATEUR

(30) Priorität: 29.07.2014 DE 102014011271
(43) Veröffentlichungstag der Anmeldung: 03.02.2016
(73) Patentinhaber: Pari GmbH, 82319 Starnberg (DE)
(72) Erfinder: Krüger, Ulf, 80799 München (DE); Jelovac, Emir, 80639 München (DE); Lohre, Birgit, 80469 München (DE)
(74) Vertreter: Müller Hoffmann & Partner

(56) Entgegenhaltungen:
- US-A- 3 405 843
- US-A- 4 592 348
- US-A1- 2002 157 664
- US-A1- 2007 074 718
- US-A1- 2012 103 326
- US-B1- 6 453 900
- US-B2- 8 695 589

## Beschreibung

Die Erfindung betrifft eine Vorschaltkammer mit einem Bedienelement für einen Inhalator.

Zur Therapierung von Patienten mit Atemwegserkrankungen ist es bekannt, den Patienten Medikamente in Form eines Aerosols zuzuführen. Das Aerosol wird häufig mit Hilfe eines Inhalators (auch als MDI - metered-dose inhaler bezeichnet) zugeführt.

Ein derartiger Inhalator weist einen das Medikament in flüssiger Form aufnehmenden Behälter auf. Am Ausgang des in einer Halterung aufgenommenen Behälters ist eine Dosiereinrichtung angeordnet, die zu einem Mundelement führt, welches vom Bediener im Anwendungsfall in den Mund genommen werden kann. Die Applikation erfolgt häufig durch den Bediener, indem er auf den Boden des Inhalators drückt, wodurch über die Dosiervorrichtung eine vorgegebene Menge des Medikaments als Aerosol in Form eines Sprühstoßes zur Verfügung gestellt wird. Das Mundelement bildet mit der üblicherweise aus Kunststoff bestehenden Halterung eine einstückige Einheit. Die Dosiereinrichtung ist auf dem Behälter aufgesetzt.

Eine bestimmungsgemäße Aufnahme des Medikaments erfordert es vom Patienten, gleichzeitig auf das Auslöseelement bzw. den Behälter des Inhalators zu drücken und gleichzeitig das Aerosol über den Mund einzuatmen. Diese Koordination von zwei Vorgängen zur gleichen Zeit kann sehr kranke Patienten, aber auch Kinder oder ältere Menschen überfordern. Dadurch ist es möglich, dass die Aerosoldosis lediglich über das Mundstück in den Mundraum des Patienten eingebracht, aber nicht in die Atemwege inhaliert wird. Die Medikamententröpfchen im Aerosol haften dann an der Mundschleimhaut und können nicht ihre vorbestimmte Wirkung erfüllen.

Zu diesem Zweck wurden Vorschaltkammern entwickelt, die zwischen dem Mundelement des Inhalators und dem Mund des Patienten eingesetzt werden können.

Eine Vorschaltkammer weist eine Gehäusekammer bzw. einen Hohlraum auf, an dem eine Anschlusseinrichtung vorgesehen ist, an der ein nicht zu der Vorschaltkammer gehörender Inhalator angesetzt werden kann. Weiterhin ist an dem Hohlraum ein Mundstück für den Patienten vorhanden. Der Patient oder auch ein Helfer kann dann zunächst den Inhalator betätigen, wodurch das Medikamenten-Aerosol in die Vorschaltkammer gelangt. In einem getrennten, zeitlich nachfolgenden Schritt kann dann der Patient das Aerosol über das Mundstück einatmen.

Mit Hilfe einer derartigen Vorschaltkammer kann somit der Inhaliervorgang zeitlich entzerrt werden. Es ist nicht mehr erforderlich, gleichzeitig den Inhalator zu betätigen und einzuatmen. Vielmehr kann zunächst der Inhalator zur Erzeugung des Aerosols betätigt werden und erst danach, unter Umständen einige Sekunden später das Aerosol eingeatmet werden.

Aus der US 7,562,656 B2 ist eine derartige Vorschaltkammer bekannt.

Obwohl sich Vorschaltkammern in der Praxis sehr gut bewährt haben, wurde festgestellt, dass die Bedienung aufgrund der Größe der Vorschaltkammern in der Regel zwei Hände benötigt. So muss die Vorschaltkammer mit einer Hand festgehalten werden, während mit der anderen Hand der Inhalator ausgelöst wird. Gerade bei Säuglingen kann dies problematisch werden, weil die Eltern das Kind mit wenigstens einer Hand festhalten müssen, so dass lediglich noch eine freie Hand zur Verfügung steht, mit der dann gleichzeitig die Vorschaltkammer an dem Mund des Kindes gehalten und der Inhalator ausgelöst werden muss.

Zu diesem Zweck wurden Vorschaltkammern entwickelt, bei denen mit Hilfe von Hebeln der Inhalator jeweils ausgelöst werden kann. Derartige Vorschaltkammern bzw. Anordnungen aus Vorschaltkammern und Inhalatoren sind zum Beispiel in US 8,695,589 B2, US 6,453,900 B1 und US 2007/0074718 A1 beschrieben.

Die bekannten Vorrichtungen sind relativ aufwändig aufgebaut und erfordern jeweils eine komplette Neuentwicklung der Vorschaltkammer. Zudem können die Inhalatoren nicht in der verkaufsüblichen Erscheinungsform, also mit Mundstück verwendet werden.

Eine weitere, besonders aufwändig gestaltete Vorschaltkammer ist aus der US 2002/0157664 A1 bekannt. Auch dort können die Inhalatoren nur ohne ihr Mundstück eingesetzt werden. Mit Hilfe eines Hebelwerks sind Sprühstöße aktivierbar, wobei das dadurch erzeugte Aerosol über die Vorschaltkammer inhaliert werden kann. Aus der US 2012/103326 A1 ist auch eine Vorschaltkammer mit Hebelwerk bekannt. Der Erfindung liegt die Aufgabe zugrunde, eine Vorschaltkammer für einen Inhalator anzugeben, die einfach aufgebaut ist, und bei der der Inhalator komplett im benutzungsfertigen Zustand eingesetzt werden kann.

Die Aufgabe wird erfindungsgemäß gelöst durch eine Vorschaltkammer mit den Merkmalen von Anspruch 1. Weiterhin wird eine Inhalationsvorrichtung mit der Vorschaltkammer und einem Inhalator angegeben. Vorteilhafte Ausgestaltungen sind in den abhängigen Ansprüchen definiert.

Eine erfindungsgemäße Vorschaltkammer für einen Inhalator weist eine Gehäusekammer auf, sowie ein an der Gehäusekammer angeordnetes Mundstück, eine an der Gehäusekammer angeordnete Anschlusseinrichtung zum Anschließen des Inhalators und eine an der Gehäusekammer gehaltene Bedieneinrichtung. Die Bedieneinrichtung ist in zwei Stellungen bewegbar und weist eine Wirkfläche auf, die derart angeordnet ist, dass die Wirkfläche in einer der Stellungen der Bedieneinrichtung auf ein Auslöseelement des Inhalators wirken kann, wenn der Inhalator an der Anschlusseinrichtung angeschlossen ist.

Das Mundstück kann an einer Seite der Gehäusekammer vorgesehen sein, während die Anschlusseinrichtung entfernt von dem Mundstück, z.B. gegenüber von dem Mundstück an der Gehäusekammer angeordnet sein kann. Wenn die Gehäusekammer eine im Wesentlichen zylindrische, rohrartige Form aufweist, kann somit das Mundstück an der einen Stirnseite und die Anschlusseinrichtung an der gegenüberliegenden Stirnseite vorgesehen sein.

Die Gehäusekammer dient als Kammer, in der das vom Inhalator kommende Aerosol "zwischengelagert" werden kann, bevor es der Patient über das Mundstück ansaugt. Der Inhalator wiederum kann seinerseits mit seinem Mundstück an der Anschlusseinrichtung befestigt werden, die an der Gehäusekammer, z.B. gegenüber von dem Mundstück angeordnet ist.

Insoweit entspricht der Aufbau der Vorschaltkammer dem Aufbau, wie er aus der US 7,562,656 B2 bekannt ist.

Darüber hinaus ist die Bedieneinrichtung vorgesehen, die in zwei Stellungen bewegbar ist. In der ersten Stellung kann die Wirkfläche auf das Auslöseelement des Inhalators drücken und dadurch einen Sprühstoß auslösen. In der zweiten Stellung drückt die Wirkfläche nicht auf das Auslöseelement, so dass die Vorrichtung in Ruhe ist.

Die Vorschaltkammer ist ohne Inhalator definiert, um die Möglichkeit zu eröffnen, den Inhalator erst bei Bedarf in die Anschlusseinrichtung einzustecken bzw. an der Anschlusseinrichtung zu befestigen. Wenn der Inhalator in der Anschlusseinrichtung in seiner bestimmungsgemäßen Position steht, befindet sich die an der Bedieneinrichtung vorgesehene Wirkfläche gegenüberliegend von dem Auslöseelement. Das Auslöseelement kann zum Beispiel der Boden des Inhalator-Behälters sein, der üblicherweise von einem Patienten mit den Fingern gedrückt wird, wenn er den Inhalator direkt betätigt.

Die Bedieneinrichtung ist direkt an der Gehäusekammer gehalten. Dadurch besteht die Möglichkeit, die Bedieneinrichtung auch nachträglich bei einer bereits vorhandenen Vorschaltkammer gemäß der US 7,562,656 B2 nachzurüsten. Weitere Veränderungen an der aus dem Stand der Technik bekannten Vorschaltkammer sind in diesem Fall nicht notwendig.

Die Anschlusseinrichtung kann ringförmig ausgebildet sein, an der Gehäusekammer lösbar befestigt sein und ausgebildet sein, um den Inhalator lösbar zu tragen. Dabei ist es insbesondere zweckmäßig, wenn die Gehäusekammer eine zylindrische, rohrförmige Grundform aufweist. Die Anschlusseinrichtung kann dann an der dem Mundstück gegenüberliegenden Stirnseite eingesteckt bzw. eingeklemmt werden.

Die Bedieneinrichtung weist eine Hebeleinrichtung auf, wobei die Hebeleinrichtung ein Hebelelement und einen das Hebelelement tragenden Hebelträger aufweist, und das Hebelelement relativ zu dem Hebelträger bewegbar, insbesondere verschwenkbar ist. Die Bewegbarkeit des Hebelelements relativ zu dem Hebelträger kann auch in Form einer Linarbewegung erfolgen. Dazu ist das Hebelelement relativ zu dem Hebelträger verschiebbar.

Dabei ist der Hebelträger an der Gehäusekammer befestigt, insbesondere lösbar befestigt. Die Befestigung erfolgt an der Gehäusekammer in der Nähe des Mundstücks so dass sich das Hebelelement weg von dem Mundstück und dem Hebelträger entlang der Mittelachse der Gehäusekammer hin zu der gegenüberliegenden Anschlusseinrichtung und damit dem Inhalator erstreckt.

Somit trägt der Hebelträger das zum Beispiel vorwiegend eine Längserstreckung aufweisende Hebelelement an der Gehäusekammer. Der Hebelträger kann unlösbar oder auch lösbar an der Gehäusekammer befestigt sein. Zum Beispiel kann der Hebelträger auf die Gehäusekammer durch Aufklicken, Aufrasten oder durch eine Steck-, eine Schraub-, eine Klebe- oder eine Kippverbindung auf der Gehäusekammer bzw. der Außenwand der Gehäusekammer befestigt werden.

Die Gehäusekammer muss dazu keine besondere Einrichtung aufweisen. Vielmehr kann der Hebelträger auch auf eine Gehäusekammer einer bereits aus dem Stand der Technik bekannten Vorschaltkammer befestigt werden, ohne dass an dieser Veränderungen vorgenommen werden müssen.

Um die Verschwenkbarkeit des Hebelelements relativ zu dem Hebelträger zu erreichen, ist es möglich, das Hebelelement federelastisch auszugestalten. Ebenso kann auch der Hebelträger elastisch sein, wobei dann der Hebel wiederrum auch steif ausgebildet sein kann, wenn die Elastizität bzw. Beweglichkeit des Hebelträgers ausreichend ist.

Ergänzend oder alternativ dazu kann zwischen dem Hebelelement und dem Hebelträger eine Gelenkeinrichtung angeordnet sein, wobei das Hebelelement aufgrund der Gelenkeinrichtung relativ zu dem Hebelträger verschwenkbar ist. Die Gelenkeinrichtung bewirkt somit einen rotatorischen Freiheitsgrad für das Hebelelement. Eine federelastische Elastizität des Hebelelements ist darüber hinaus möglich.

Die Gelenkeinrichtung kann als Gelenk eine Drehachse bereitstellen. Sie kann auch zum Beispiel als Filmscharnier verwirklicht werden.

Bei einer Ausführungsform kann das Hebelelement eine längliche Erstreckung aufweisen und an einem trägerseitigen Ende an dem Hebelträger gehalten sein. An dem anderen, dem Hebelträger gegenüberliegenden inhalatorseitigen Ende des Hebelelements kann die Wirkfläche ausgebildet sein.

Bei einer Variante kann eine Sicherungseinrichtung vorgesehen sein, zum Abstützen des Inhalators an wenigstens einer Seite, um ein Verdrehen des Inhalators um eine Mittelachse der Gehäusekammer relativ zu der Aufnahmeeinrichtung zu verhindern. Für den ordnungsgemäßen Betrieb des Inhalators ist es notwendig, dass der Inhalator zuverlässig von der Anschlusseinrichtung gehalten wird. Durch ein Verdrehen des Inhalators relativ zu der Aufnahmeeinrichtung besteht die Gefahr, dass der Inhalator aus der Aufnahmeeinrichtung herausfällt. Dies kann insbesondere bei einem Transport des Inhalators mit der Vorschaltkammer geschehen. Mit Hilfe der Sicherungseinrichtung wird ein ungewolltes Verdrehen des Inhalators vermieden, da der Inhalator relativ zu der Aufnahmeeinrichtung in seiner Position gehalten wird.

Bei einer Ausführungsform kann an dem inhalatorseitigen Ende des Hebelelements eine Abstützeinrichtung vorgesehen sein, zum Abstützen des Inhalators an seiner rückwärtigen, von der Lage des Hebelelements abgewandten Seite. Es wurde festgestellt, dass bei einem Betätigen des Hebelelements die Gefahr besteht, dass aufgrund von durch das Hebelelement ausgeübten schräg wirkenden Kräften der Inhalator bzw. der Behälter des Inhalators nach hinten, von der Vorschaltkammer weg gebogen wird. Dadurch kann der Inhalator aus der Anschlusseinrichtung herausgedrückt werden. Um dies zu verhindern, kann die Abstützeinrichtung vorgesehen werden, mit der der Inhalator rückwärtig, also auf der von der Vorschaltkammer abgewandten Seite abgestützt wird. Dadurch wird der Inhalator zuverlässig in der Anschlusseinrichtung gehalten.

Die Sicherungseinrichtung oder auch die Abstützeinrichtung können jeweils eine Fläche aufweisen, die an dem inhalatorseitigen Ende des Hebelelements ausgebildet ist. Das bedeutet, dass das Hebelelement an dem inhalatorseitigen Ende verlängert ist, um die erforderlichen Flächen für die oben beschriebene Sicherungseinrichtung und/oder die Abstützeinrichtung bereitzustellen. Eine weitere Veränderung der Vorschaltkammer ist dabei nicht erforderlich, so dass - wie oben bereits erwähnt - auch für die Gehäusekammer eine Gehäusekammer genutzt werden kann, wie sie aus dem Stand der Technik bei Vorschaltkammern bekannt ist, auf die dann mit Hilfe des Hebelträgers ein entsprechend geformtes Hebelelement mit den diversen Flächen aufgesetzt werden kann.

Die Fläche an der Sicherungseinrichtung bzw. die entsprechende Fläche an der Abstützeinrichtung dient dazu, einen Anschlag für das Halten bzw. Abstützen des Inhalators bereitzustellen. Sie kann als Teil des Hebelelements ausgebildet sein, also einstückig mit dem Hebelelement verbunden sein. Zur Ausbildung der Fläche genügt es, wenn sie auch nur einen geringen linien- oder ringausschnittförmigen Kontakt mit dem Inhalator ermöglicht.

Bei einer Ausführungsform kann eine Verriegelungseinrichtung vorgesehen sein, zum Verriegeln der Position des Hebelelements in einer der Stellungen. Wie oben ausgeführt, kann das Hebelelement als Teil der Bedieneinrichtung in wenigstens zwei Stellen bewegbar sein, nämlich in die erste Stellung, in der die Wirkfläche auf das Auslöseelement des Inhalators einwirkt, zum Beispiel drückt, sowie in die zweite Stellung, in der die Wirkfläche nicht auf das Auslöseelement drückt. In wenigstens einer dieser Stellungen kann das Hebelelement verriegelt werden, um zum Beispiel beim Transport der Vorschaltkammer ein ungewolltes Auslösen des Inhalators zu verhindern.

Dementsprechend kann die Verriegelungseinrichtung zwischen zwei Zuständen bewegbar sein, nämlich einem Verriegelungszustand und einem Betriebszustand, wobei die Verriegelungseinrichtung in dem Verriegelungszustand zum Halten des Hebelelements in einer vorgegebenen Stellung dient und in dem Betriebszustand eine Bewegung des Hebelelements freigibt. Der Verriegelungszustand kann dabei je nach Ausführungsform die erste oder die zweite Stellung des Hebelelements sein. Das bedeutet, dass das Hebelelement auch in einer Stellung verriegelt werden kann, in der es dauerhaft auf das Auslöseelement drückt. Dies ist unproblematisch, da das Auslöseelement nur bei einem Betätigen einmalig den Ausstoß von Aerosol bewirkt. Bleibt das Auslöseelement danach in der gedrückten Stellung, wird kein weiteres Aerosol produziert. Es kann auch kein Medikament oder Treibgas aus dem Behälter des Inhalators austreten.

Die Hebeleinrichtung kann einen Griffbereich aufweisen, zum Greifen durch einen Bediener. Dabei ist es möglich, die Lage der auf das Auslöseelement wirkenden Wirkfläche relativ zu dem Griffbereich zu verändern. Der Griffbereich kann zum Beispiel die Oberseite des Hebelelements sein und durch den Bediener mit der Hand gedrückt werden.

Die Veränderung der Lage der Wirkfläche relativ zu dem Griffbereich kann zweckmäßig sein, um die Vorrichtung an unterschiedlich große Inhalatoren anzupassen. So ist es möglich, dass das Auslöseelement an verschiedenen Stellen bzw. in verschiedenen Höhenniveaus zu liegen kommt, so dass der Abstand zwischen der Wirkfläche und dem Auslöseelement variiert. Um die Vorrichtung daran anzupassen, kann die Lage der Wirkfläche verändert werden, zum Beispiel durch Verschieben oder Verschrauben des Elements, an dem die Wirkfläche vorgesehen ist. Insbesondere kann dazu das Hebelelement in wenigstens zwei Teile aufgeteilt werden, deren Abstand zueinander einstellbar variabel ist.

Die Hebellänge kann ebenfalls variabel sein, um die Vorrichtung und insbesondere die Bedieneinrichtung an unterschiedliche Behältergrößen des Inhalators anzupassen (Länge, Höhe). Ebenso ist es möglich, verschiedene Lagerungs- oder Befestigungspunkte des Hebelelements an der Gehäusekammer bzw. dem Hebelträger vorzusehen.

Die Anschlusseinrichtung kann an einer Stirnseite der Gehäusekammer gehalten sein und an dieser Stirnseite in wenigstens drei Stellungen verdrehbar sein, nämlich in eine erste Drehstellung, in der der Inhalator in der Anschlusseinrichtung festgehalten ist und die Anschlusseinrichtung in der Gehäusekammer festgehalten ist, in eine zweite Drehstellung, in der der Inhalator aus der Anschlusseinrichtung entfernbar ist und die Anschlusseinrichtung in der Gehäusekammer festgehalten ist, sowie in eine dritte Drehstellung, in der die Anschlusseinrichtung von der Stirnseite der Gehäusekammer entfernbar ist.

In der ersten Drehstellung wird somit der Inhalator in der Anschlusseinrichtung gehalten und ist insoweit betriebsbereit. In der zweiten Drehstellung kann der Inhalator ausgewechselt werden, ohne dass die Anschlusseinrichtung von der Gehäusekammer entfernbar ist. Vielmehr bleibt die Anschlusseinrichtung an der Gehäusekammer festgehalten. Erst in der dritten Drehstellung kann dann auch die Anschlusseinrichtung von der Gehäusekammer abgenommen werden, um zum Beispiel freien Zugang zu der Gehäusekammer zu Reinigungszwecken zu erhalten. Die verschiedenen Drehstellungen können z.B. durch Bajonettverschlüsse realisiert werden, so dass der Anschlussring dabei mit Hilfe eines Bajonettverschlusses an der Gehäusekammer befestigt sein kann.

Die somit beschriebene Vorschaltkammer kann insbesondere in einer Inhalationsvorrichtung mit einem Inhalator verwendet werden, wobei der Inhalator einen ein Medikament aufnehmenden Behälter aufweist, sowie ein an dem Behälter angeordnetes Mundelement und eine wirkungsmäßig zwischen dem Behälter und dem Mundelement angeordnete Dosiereinrichtung zum Erzeugen eines Aerosols, wobei das Aerosol eine vorbestimmte Menge des Medikaments enthält. Der Inhalator kann mit seinem Mundelement an der Anschlusseinrichtung befestigbar sein, wobei das Auslöseelement des Inhalators eine Bodenfläche des Behälters ist.

Bei dem Inhalator handelt es sich somit um einen üblichen Inhalator, mit dem in der Regel Medikamente zur Therapie von Atemwegserkrankungen o.ä. verabreicht werden. Ein derartiger Inhalator ist auch als MDI (metered-dose inhaler) vielfältig bekannt.

Diese und weitere Merkmale werden nachfolgend anhand von Beispielen unter Zuhilfenahme der begleitenden Figuren näher erläutert. Es zeigen:
- Fig. 1: eine Inhalationsvorrichtung mit einer Vorschaltkammer und einem Inhalator in Seitenansicht,
- Fig. 2a: eine Teildarstellung einer Inhalationsvorrichtung in Seitenansicht;
- Fig. 2b: die Inhalationsvorrichtung von Fig. 2a in der Draufsicht;
- Fig. 3a: eine Vorschaltkammer in Seitenansicht;
- Fig. 3b: eine Teildarstellung der Vorschaltkammer von Fig. 3a mit aufgesetzter Hebeleinrichtung;
- Fig. 4: eine Teildarstellung einer Vorschaltkammer in Seitenansicht;
- Fig. 5: eine Teildarstellung einer Inhalationsvorrichtung in Seitenansicht;
- Fig. 6: eine Detailansicht in der Draufsicht;
- Fig. 7: Varianten eines inhalatorseitigen Hebelendes;
- Fig. 8: eine Draufsicht auf ein inhalatorseitiges Ende einer Vorschaltkammer;
- Fig. 9a: eine Rückansicht einer Inhalationsvorrichtung;
- Fig. 9b: die Inhalationsvorrichtung von Fig. 9a mit verdrehtem Inhalator und verdrehter Anschlusseinrichtung;
- Fig. 10: eine Seitenansicht einer nicht-erfindungsgemäßen Variante einer Inhalationsvorrichtung;
- Fig. 11: eine weitere nicht-erfindungsgemäße Variante einer Inhalationsvorrichtung in Teildarstellung;
- Fig. 12: eine perspektivische Teilansicht einer Vorschaltkammer mit Verriegelungseinrichtung;
- Fig. 13: eine schematische Teilansicht einer Inhalationsvorrichtung mit Verriegelungseinrichtung;
- Fig. 14: eine Teilansicht einer Inhalationsvorrichtung für Inhalatoren unterschiedlicher Größe.

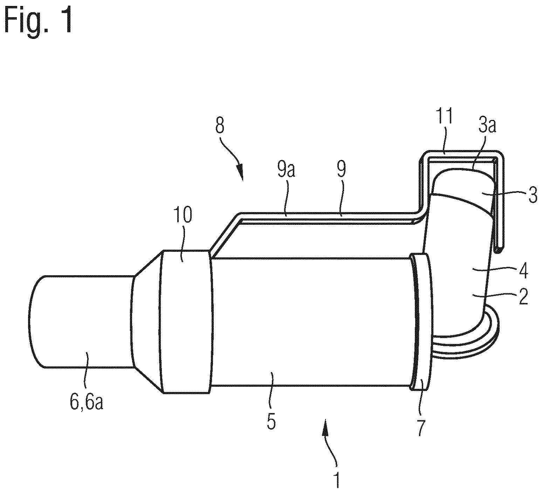

Fig. 1 zeigt in Seitenansicht eine Inhalationsvorrichtung mit einer Vorschaltkammer 1 und einem Inhalator 2.

Bei dem Inhalator 2 handelt es sich um einen handelsüblichen Inhalator (auch als MDI - metered-dose inhaler bezeichnet), mit dem ein Medikament in vorbestimmter Dosierung in ein Aerosol gewandelt wird, welches über ein in Fig. 1 nicht sichtbares Mundstück von einem Patienten inhaliert werden kann. Ein das Medikament und ein Treibgas enthaltender Behälter 3 ist in eine Halterung 4 eingesetzt, insbesondere eingeschraubt. An der Halterung 4 ist auch das (nicht sichtbare) Mundelement einstückig ausgebildet.

Da der Aufbau eines derartigen Inhalators aus dem Stand der Technik vielfältig bekannt ist, erübrigt sich an dieser Stelle eine weitere Erläuterung.

Durch Drücken des obenliegenden Bodens 3a des mit seinem Auslass nach unten in der Halterung 4 gehaltenen Behälters 3 wird ein Sprühstoß ausgelöst und dem Mundelement (in Fig. 1 links unten an der Halterung 4, jedoch nicht sichtbar) zugeführt. Zum Bewirken des Sprühstoßes muss daher der Patient mit einiger Kraft auf den Boden 3a des Behälters 3 drücken.

Die Vorschaltkammer 1 weist eine Gehäusekammer 5 auf, die im Wesentlichen einem hohlzylindrischen Rohr entspricht und an den Stirnseiten abgedeckt ist. An einer Stirnseite ist ein Mundstück 6 vorgesehen, das zum Beispiel mit einem Schnabelventil versehen sein kann. In Fig. 1 ist das Mundstück 6 durch eine Schutzabdeckung 6a abgedeckt. An dem gegenüberliegenden stirnseitigen Ende der Gehäusekammer 5 ist eine Anschlusseinrichtung 7 aufgesetzt, die einerseits die Gehäusekammer 5 deckelartig verschließt und andererseits die Anschlussmöglichkeit zum Anschließen des Inhalators 2 bildet.

In der Anschlusseinrichtung 7 ist eine in Fig. 1 nicht sichtbare Öffnung ausgebildet, in die das Mundelement des Inhalators 2 eingesteckt werden kann. Der Inhalator 2 wird auf diese Weise sicher an der Gehäusekammer 5 gehalten.

Der Aufbau einer derartigen Vorschaltkammer 1 ist zum Beispiel in der US 7,562,656 B2 beschrieben. Zur Vermeidung von Wiederholungen wird daher auf diese Schrift Bezug genommen.

An der Außenseite der Gehäusekammer 5 ist eine als Bedieneinrichtung dienende Hebeleinrichtung 8 aufgesetzt. Die Hebeleinrichtung 8 weist einen als Hebelelement dienenden Hebel 9 auf, der von einem Hebelträger 10 an der Gehäusekammer 5 gehalten wird.

Der Hebel 9 ist derart ausgebildet, dass er sich von dem in der Nähe des Mundstücks 6 angeordneten Hebelträger 10 entlang der Mittelachse der Gehäusekammer 5 nach hinten in Richtung des Inhalators 2 weg erstreckt und dort den Boden 3a des Behälters 3 des Inhalators 2 umschließt.

Der Hebel 9 wird über den Hebelträger 10 an der Gehäusekammer 5 beweglich gehalten, ist also relativ zu dem Hebelträger 10 bewegbar bzw. verschwenkbar. Zu diesem Zweck weisen der Hebel 9 und der Hebelträger 10 eine gewisse Beweglichkeit auf, die es insbesondere ermöglicht, dass das inhalatorseitige Ende des Hebels 9 derart bewegt werden kann, dass eine an dem Ende ausgebildete Wirkfläche 11 auf den Boden 3a des Behälters 3 drücken kann, um einen Sprühstoß auszulösen. Ein Bediener der Inhalationsvorrichtung kann somit die Gehäusekammer 5 mit dem Hebel 9 mit einer Hand halten und den Hebel 9 in Richtung der Gehäusekammer 5 verschwenken. Der Bediener kann dabei auf einen Griffbereich 9a des Hebels 9 drücken. Dadurch wird die Wirkfläche 11 verlagert und gegen den Boden 3a des Inhalatorbehälters 3 gedrückt, wodurch ein Sprühstoß ausgelöst werden kann. Das Aerosol aus dem Behälter 3 tritt über das Mundelement des Inhalators 2 und die Anschlusseinrichtung 7 in den Innenraum der Gehäusekammer 5 und kann dort von dem Patienten inhaliert werden.

Für die Beweglichkeit des Hebels 9 kommt es vor allem darauf an, dass die Wirkfläche 11 in Richtung des Bodens 3a bewegt werden kann. Dementsprechend sind für die Ausgestaltung des Hebels 9 und des Hebelträgers 10 verschiedene Lösungen möglich, um diese Beweglichkeit zu erreichen.

So kann zum Beispiel der Hebelträger 10 elastisch sein und den relativ starren Hebel 9 halten, der dann durch seine Anbindung beweglich ist. Ebenso ist es möglich, den Hebelträger 10 eher starr auszuführen und den Hebel 9 selbst elastisch auszubilden. Aufgrund der Länge des Hebels 9 kann - trotz des mundstückseitig fixierten Endes des Hebels 9 die gewünschte Beweglichkeit der am inhalatorseitigen Ende vorgesehenen Wirkfläche 11 erreicht werden.

Ebenso ist es möglich, dass zwischen dem Hebelträger 10 und dem Hebel 9 eine Gelenkeinrichtung ausgebildet ist, um die Bewegbarkeit des Hebels 9 zu erreichen. Die Gelenkeinrichtung kann als Gelenk mit einem zusätzlichen Achselement ausgeführt werden. Ebenso kann die Gelenkeinrichtung auch als einfaches Filmscharnier ausgebildet sein.

Der Hebelträger 10 kann mit der Gehäusekammer 5 fest verbunden sein. So ist es möglich, den Hebelträger 10 auf die Außenwand der Gehäusekammer 5 z.B. aufzuschrumpfen oder aufzukleben. Ebenso kann der Hebelträger 10, der zum Beispiel ringförmig ausgebildet ist und damit die Gehäusekammer 5 umschließt, in Form einer Schelle gestaltet sein, die auf der Gehäusekammer 5 festgeschraubt wird.

Bei einer Variante wird der Hebelträger 10 lediglich auf die Gehäusekammer 5 aufgeclipst oder aufgeschoben. Der Hebelträger 10 kann auch in eine Tasche oder einen Ring eingeschoben werden, die an der Gehäusekammer 5 vorgesehen ist.

An der Außenwand der Gehäusekammer 5 müssen keine besonderen Vorkehrungen getroffen werden, um die Befestigung des Hebelträgers 10 zu ermöglichen. Es ist jedoch auch möglich, zum Beispiel eine umlaufende Nut an der Gehäusekammer 5 vorzusehen, in der der ring- oder spangenförmige Hebelträger 10 gehalten wird.

Nachfolgend werden weitere Varianten beschrieben.

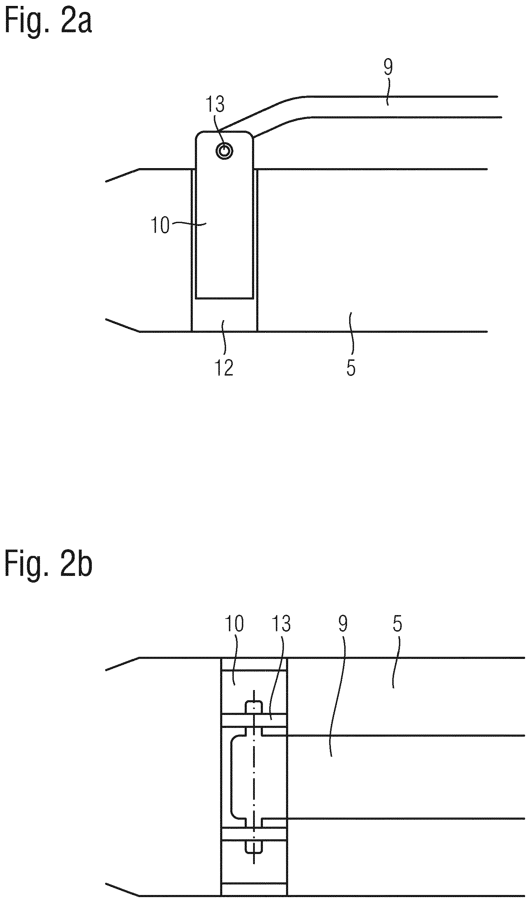

Fig. 2a zeigt eine Variante für die Befestigung des Hebels 9 bzw. des Hebelträgers 10 in Seitenansicht und Fig. 2b zeigt die Anordnung von Fig. 2a in der Draufsicht.

In der Außenwand der Gehäusekammer 5 ist eine umlaufende Ausnehmung 12 in Form einer Nut, einer Fuge oder eines Einstichs vorgesehen, in die der Hebelträger 10 eingesetzt ist. Der Hebelträger 10 ist im gezeigten Beispiel als offene Spange dargestellt, die in die Ausnehmung 12 eingeclipst wird. Alternativ wäre es möglich, den Hebelträger 10 auch als geschlossenen Ring oder als Schelle auszuführen.

Am Übergang zwischen dem Hebelträger 10 und dem Hebel 9 ist eine Gelenkeinrichtung 13 in Form eines Drehlagers vorgesehen. Mit Hilfe der Gelenkeinrichtung 13 ist einfach möglich, den Hebel 9 relativ zu dem Hebelträger 10 und der Gehäusekammer 5 zu verschwenken.

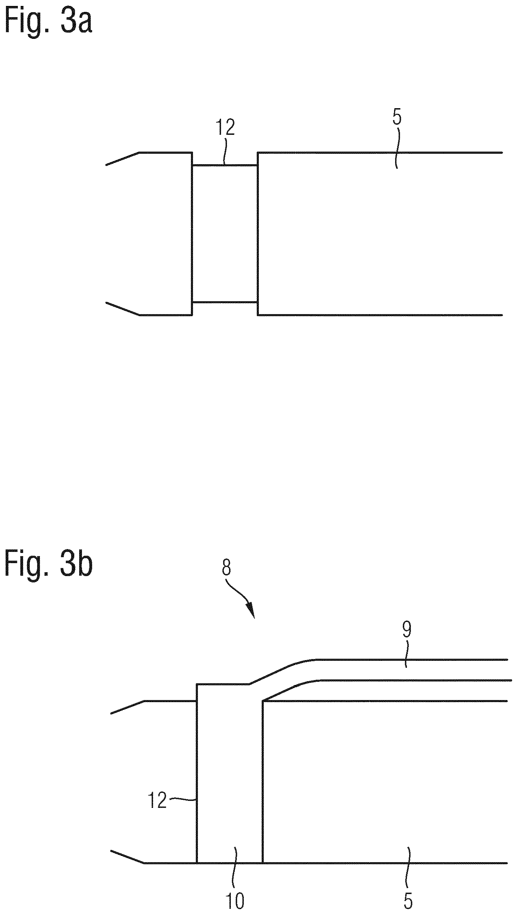

In Fig. 3a wird die Gehäusekammer 5 mit der Ausnehmung 12 dargestellt, jedoch ohne den Hebelträger 10.

In Fig. 3b ist eine vereinfachte Anordnung gezeigt, bei der der Hebelträger 10 ebenfalls in Form eines Montageclips in die Ausnehmung 12 eingesetzt ist. Mit dem Hebelträger 10 ist der Hebel 9 verbunden. Da der Hebel 9 eine gewisse Elastizität, insbesondere Federelastizität aufweist, ist keine zusätzliche Gelenkeinrichtung erforderlich, um die Beweglichkeit des Hebels 9 zu erreichen.

Insbesondere können der Hebel 9 und der Hebelträger 10 in Form der gesamten Hebeleinrichtung 8 einstückig, zum Beispiel aus Kunststoff oder aus Metall hergestellt werden.

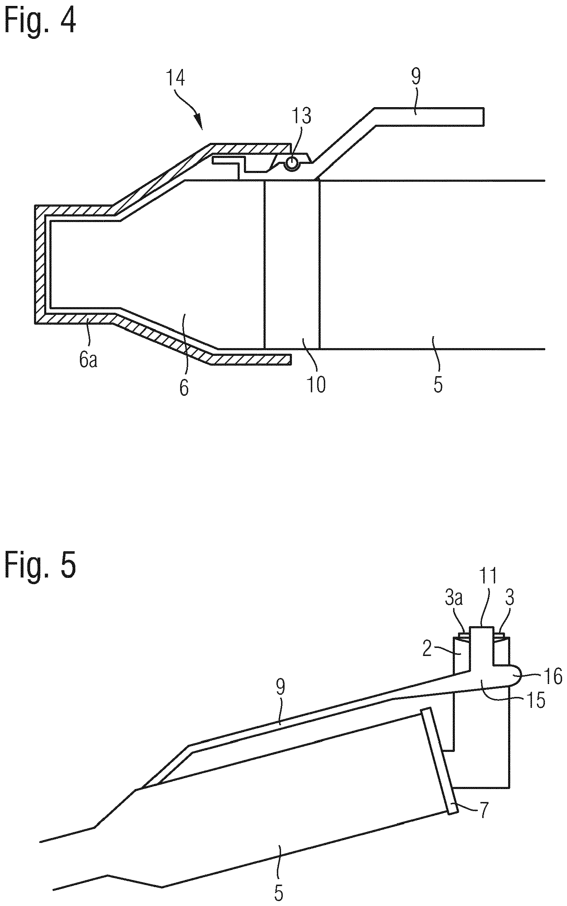

Fig. 4 zeigt eine weitere Variante, bei der der Hebelträger 10 und der Hebel 9 eher starr ausgeführt sind und der Hebel 9 bezüglich des Hebelträgers 10 aufgrund der dazwischen angeordneten Gelenkeinrichtung 13 verschwenkt werden kann. Weiterhin ist eine Transportsicherung 14 vorgesehen. Die Transportsicherung 14 kann in die Schutzabdeckung 6a für das Mundstück 6 integriert sein, also Teil der Schutzabdeckung 6a sein, oder auch in das Mundstück 6 integriert sein. Die Wirkung der Transportsicherung 14 wird dadurch erreicht, dass der Hebel 9 über die Gelenkeinrichtung 13 hinaus in Richtung des Mundstücks 6 verlängert ist und mit dieser Verlängerung unter die Schutzabdeckung 6a bzw. Transportsicherung 14 greift. Wenn daher die z.B. als Kappe ausgebildete Schutzabdeckung 6a montiert ist, wie in Fig. 4 gezeigt, übergreift die Schutzabdeckung 6a als Transportsicherung 14 das verlängerte Ende des Hebels 9 und blockiert seine Bewegung.

Fig. 5 zeigt eine weitere Variante, wobei jetzt die Gestaltung des inhalatorseitigen Endes des Hebels 9 betrachtet werden soll.

Wie in Fig. 5 erkennbar, ist die Wirkfläche 11 an einem Bügel ausgebildet, der den Boden 3a des Behälters 3 überragt. Der Hebel 9 selbst bildet eine seitliche Stützfläche 15, die als Sicherungseinrichtung dient und den Inhalator 2 in der Betriebsstellung abstützt. Dadurch wird verhindert, dass der Inhalator 2 relativ zu der Gehäusekammer 5 unerwünscht verdreht werden kann. Der Inhalator 2 wird durch die Gestaltung des Ende des Hebels 9 in der bestimmungsgemäßen Betriebsstellung gehalten.

Die seitliche Stützfläche 15 geht in eine rückwärtige Abstützfläche 16 über, die als Abstützeinrichtung dient. Beim Betätigen des Hebels 9 wird nämlich bei der gezeigten Ausführungsform eine Kraft auf den Inhalator 2 ausgeübt, wodurch der Inhalator 2 die Tendenz hat, aus der Anschlusseinrichtung 7 herausgedrückt zu werden. Um den Inhalator 2 zuverlässig in der Anschlusseinrichtung 7 zu halten, ist die rückwärtige Abstützfläche 16 vorgesehen. Ein Ausweichen des Inhalators 2 nach hinten, weg von der Gehäusekammer 5 und der Anschlusseinrichtung 7 wird dadurch verhindert. Wenn durch den Hebel 9 hingegen keine Kraft auf den Inhalator 2 ausgeübt wird, die den Inhalator 2 aus der Anschlusseinrichtung 7 drücken könnte, ist die Abstützfunktion der Abstützfläche 16 nicht erforderlich.

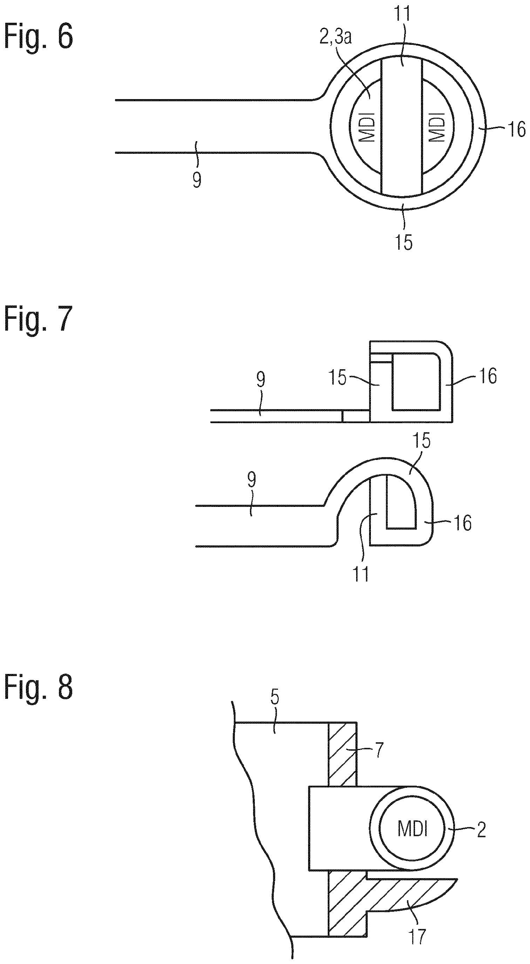

Fig. 6 zeigt eine Variante des inhalatorseitigen Endes des Hebels 9 in der Draufsicht.

Dabei umschließt die seitliche Stützfläche 15 den Inhalator 2 ringförmig. Der Boden 3a des Inhalators 2 wird durch die bogenförmige Wirkfläche 11 abgedeckt.

Fig. 7 zeigt weitere Varianten für die Gestaltung des inhalatorseitigen Endes des Hebels 9 in Drauf- und Seitenansicht. Hier umfasst die seitliche Stützfläche 15 den Inhalator 2 nur teilweise und ermöglicht so das Schwenken des Inhalators 2 um die Mittel- bzw. Längsachse der Gehäusekammer 5.

Die seitliche Stützfläche 15 kann übergangslos in die rückwärtige Abstützfläche 16 übergehen, wie zum Beispiel die Fig. 6 und 7 zeigen.

Fig. 8 zeigt eine Variante, bei der eine seitliche Stützfläche 17 nicht am Ende des Hebels 9, sondern an der Anschlusseinrichtung 7 ausgebildet ist. Aus der Darstellung von Fig. 8 in der Draufsicht ist erkennbar, dass der Inhalator 2 nicht beliebig relativ zu der Gehäusekammer 5 bzw. der Anschlusseinrichtung 7 verdreht werden kann, weil er durch die seitliche Stützfläche 17 in der senkrechten Position gehalten wird. Ausführungsformen mit zwei seitlichen Stützflächen können ebenfalls vorteilhaft sein.

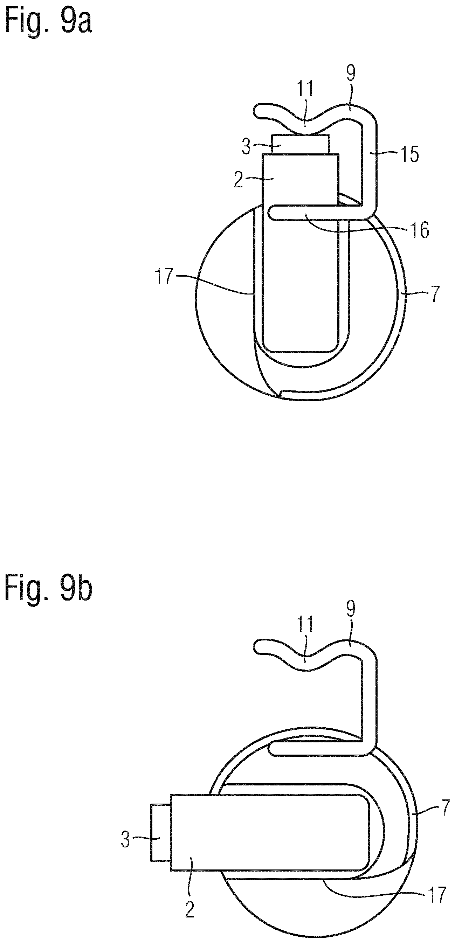

Fig. 9a zeigt eine rückwärtige Ansicht einer weiteren Variante in Betriebsstellung. Fig. 9b zeigt die Variante in einer Stellung, in der der Inhalator 2 entnommen werden kann.

Der Inhalator 2 wird, ähnlich wie bei den Varianten in den Fig. 5 bis 7 gezeigt, durch das Ende des Hebels 9 teilweise umschlossen, so dass die Wirkfläche 11 gegenüber von dem Boden 3a des Behälters 3 zu liegen kommt, die seitliche Stützfläche 15 ein Verdrehen des Inhalators 2 in einer Richtung verhindert und die rückwärtige Abstützfläche 16 ein Ausweichen des Inhalators 2 weg von der Gehäusekammer 5 blockiert.

Darüber hinaus ist an der ringförmigen Anschlusseinrichtung 7 die seitliche Stützfläche 17 vorgesehen, die ein Verdrehen des Inhalators 2 in einer zu der seitlichen Stützfläche 15 entgegengesetzten Richtung blockiert.

Nur durch ein Verdrehen zusammen mit der Anschlusseinrichtung 7 kann der Inhalator 2 freigegeben werden, wie Fig. 9b zeigt.

Ergänzend ist es möglich, die Anschlusseinrichtung 7 in insgesamt drei Stellungen zu verdrehen, nämlich in die Stellungen nach den Fig. 9a und 9b sowie in einer weiteren Stellung (ausgehend von Fig. 9b um 90° entgegen dem Uhrzeigersinn). In letzterer Stellung kann dann die Anschlusseinrichtung von der Gehäusekammer 5 abgenommen werden, um zum Beispiel freien Zugang zu der Gehäusekammer 5 für Reinigungszwecke zu erhalten. Dabei kann es zweckmäßig sein, wenn die Anschlusseinrichtung 7 durch einen Bajonettverschluss an der Gehäusekammer 5 befestigt ist.

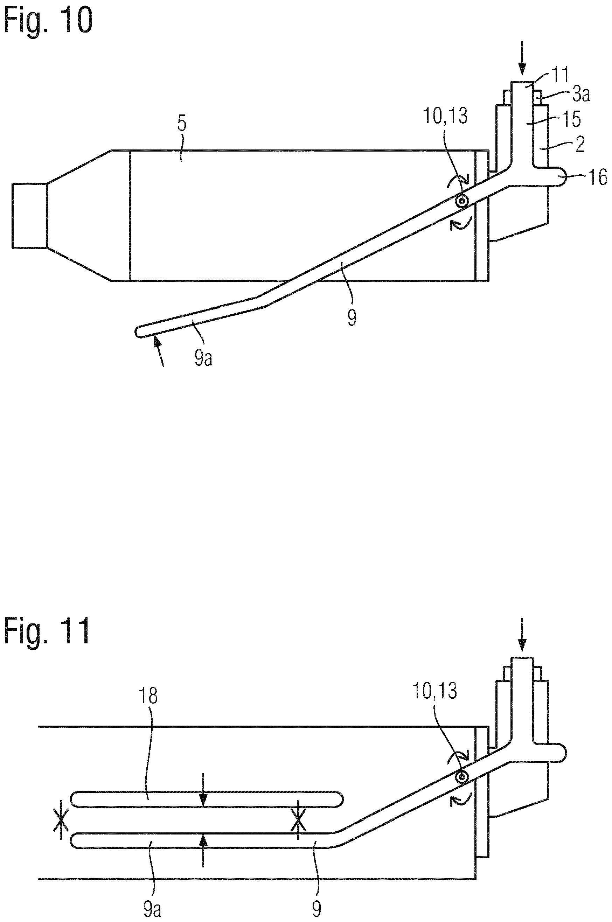

Fig. 10 zeigt eine weitere nicht-erfindungsgemäße Variante, bei der der Hebelträger 10 an dem inhalatorseitigen Ende der Gehäusekammer 5 vorgesehen ist. Dort ist auch die Gelenkeinrichtung 13 angeordnet, die ein Verschwenken des Hebels 9 relativ zu der Gehäusekammer 5 ermöglicht.

In diesem Fall muss der Bediener - in Fig. 10 von unten - gegen einen Griffbereich 9a des Hebels 9 drücken (in Pfeilrichtung), um einen Sprühstoß auszulösen. Durch das Drücken des Griffbereichs 9a in Richtung der Gehäusekammer 5 senkt sich die gegenüberliegende Wirkfläche 11 ab und betätigt den Behälterboden 3a.

Fig. 11 zeigt eine weitere nicht-erfindungsgemäße Variante, bei der zusätzlich zu dem Hebel 9 ein Griff 18 an der Gehäusekammer 5 vorgesehen ist, um ein Abstützen der Hand des Bedieners zu ermöglichen.

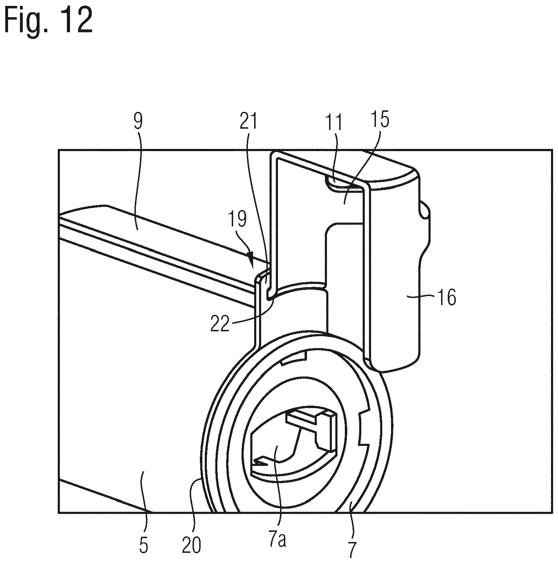

Fig. 12 zeigt eine Ausführungsform mit einer Verriegelungseinrichtung 19. Bei einem Transport der Inhalationsvorrichtung besteht die Gefahr, dass zum Beispiel in einer Handtasche versehentlich Druck auf den Hebel 9 ausgeübt und damit ein ungewollter Sprühstoß ausgelöst wird. Zu diesem Zweck ist die Verriegelungseinrichtung 19 vorgesehen, die eine ungewollte Bewegung des Hebels 9 verhindern soll.

Die Verriegelungseinrichtung 19 weist einen die Gehäusekammer 5 umschließenden Verriegelungsring 20 mit einem Verriegelungsfortsatz 21 auf. In dem Verriegelungsfortsatz 21 ist eine Ausnehmung 22 vorgesehen, die über den Hebel 9 in die in Fig. 12 gezeigte Stellung geführt werden kann. Dadurch wird der Hebel 9 in der gezeigten Stellung blockiert. Insbesondere kann die Wirkfläche 11 nicht auf den Inhalator 2 drücken (Verriegelungszustand).

Zum Lösen des Hebels 9 wird der Verriegelungsring 20 mit dem Verriegelungsfortsatz 21 verschwenkt. Der Hebel 9 gleitet damit aus der Ausnehmung 20 und ist frei beweglich (Betriebszustand).

In Fig. 12 ist darüber hinaus auch gut die Gestaltung der ringförmigen Anschlusseinrichtung 7 erkennbar, mit einer Öffnung 7a, in der ein Mundelement des Inhalators 2 eingeschoben werden kann.

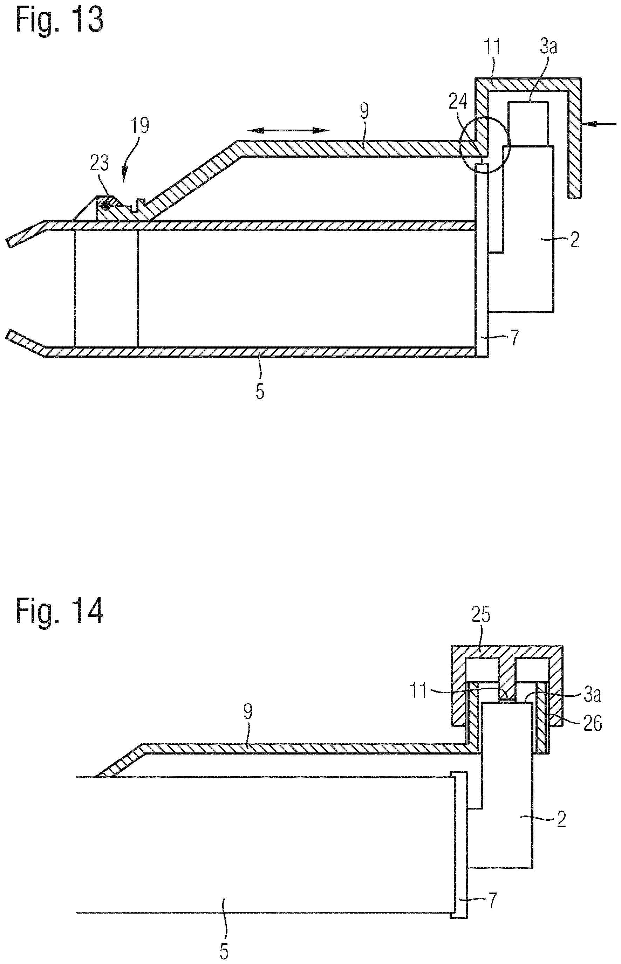

Fig. 13 zeigt ein anderes Beispiel für eine mögliche Verriegelungseinrichtung 19. In dem in Fig. 13 dargestellten Verriegelungszustand ist der Hebel 9 relativ zu der Gehäusekammer 5 axial in zwei Verrastungsstufen bewegbar. Die Verrastungsstufen werden durch eine Rasteinrichtung 23 bestimmt. In der in Fig. 13 gezeigten ersten Verrastungsstufe befindet sich ein Teil des Hebels 9 direkt über einem Anschlag 24. Der Hebel 9 kann somit nur geringfügig nach unten in Richtung des Inhalators 2 bewegt werden. Ein versehentliches Auslösen eines Sprühstoßes wird damit verhindert.

Bei axialem Verschieben des Hebels 9 in die zweite Verraststufe (in Fig. 13 nach links) wird der Hebel 9 derart bewegt, dass er nicht mehr gegen den Anschlag 24 anschlagen kann. Dadurch ist der Hebel 9 frei nach unten bewegbar und die Wirkfläche 11 kann auf den Boden 3a des Inhalators 2 drücken.

Fig. 14 zeigt eine Variante, bei der die Inhalationsvorrichtung an unterschiedliche Größen von Inhalatoren 2 anpassbar ist.

So gibt es Inhalatoren mit unterschiedlichen Behältergrößen, wobei insbesondere die Länge, also axiale Erstreckung des Behälters 3 und damit die Lage des Behälterbodens 3a veränderlich ist.

Um den Hebel 9 und die Lage der Wirkfläche 11 an die unterschiedlichen geometrischen Gegebenheiten anpassen zu können, ist die Lage der Wirkfläche 11 relativ zu dem restlichen Hebel 9 veränderbar. Dies wird in dem gezeigten Beispiel dadurch erreicht, dass die Wirkfläche 11 an einem relativ zu dem Hebel 9 verschraubbaren Deckel 25 ausgebildet ist. Je nach Axialstellung des Deckels 25 befindet sich die Wirkfläche 11 in unterschiedlicher Höhe bezüglich des Hebels 9 und des Inhalators 2.

Vorzugsweise lässt sich die Position des Deckels 25 fixieren, zum Beispiel durch ein verhältnismäßig schwergängiges Gewinde 26 zwischen dem Ende des Hebels 9 und dem Deckel 25.

Der Deckel 25 kann bei einer Variante auch als Verschluss für das Mundstück 6 dienen, wenn die Vorschaltkammer nicht benutzt wird.

Alternativ dazu ist es möglich, den Deckel 25 in unterschiedlichen Axialstellungen zu verrasten.

Die anhand der verschiedenen Ausführungsformen erläuterten Einzelmerkmale lassen sich beliebig kombinieren, so dass die Vorschaltkammer je nach Wunsch ausgestaltet werden kann.

## Patentansprüche

1. Vorschaltkammer (1) für einen Inhalator (2), mit
- einer Gehäusekammer (5),
- einem an der Gehäusekammer (5) angeordneten Mundstück (6),
- einer an der Gehäusekammer (5) angeordneten Anschlusseinrichtung (7) zum Anschließen eines an dem Inhalator (2) vorgesehenen Mundelements, und mit
- einer an der Gehäusekammer (5) gehaltenen Bedieneinrichtung, die in zwei Stellungen bewegbar ist und eine Wirkfläche (11) aufweist, die derart angeordnet ist, dass die Wirkfläche (11) in einer der Stellungen der Bedieneinrichtung auf ein Auslöseelement (3a) des Inhalators wirken (2) kann, wenn der Inhalator (2) an der Anschlusseinrichtung (7) angeschlossen ist,
wobei
- die Bedieneinrichtung eine Hebeleinrichtung (8) aufweist,
- die Hebeleinrichtung (8) ein Hebelelement (9) und einen das Hebelelement (9) tragenden Hebelträger (10) aufweist, und wobei
- das Hebelelement (9) relativ zu dem Hebelträger (10) bewegbar, insbesondere verschwenkbar ist, **dadurch gekennzeichnet, dass** der Hebelträger (10) an der Gehäusekammer (5) in der Nähe des Mundstücks (6) befestigt ist.

2. Vorschaltkammer nach Anspruch 1, wobei die Anschlusseinrichtung (7)
- ringförmig ausgebildet ist,
- an der Gehäusekammer (5) lösbar befestigbar ist, und
- ausgebildet ist, um den Inhalator (2) lösbar zu tragen.

3. Vorschaltkammer nach einem der vorstehenden Ansprüche, wobei der Hebelträger (10) an der Gehäusekammer (5) befestigt, insbesondere lösbar befestigt ist.

4. Vorschaltkammer nach einem der vorstehenden Ansprüche, wobei
- zwischen dem Hebelelement (9) und dem Hebelträger (10) eine Gelenkeinrichtung (13) angeordnet ist,
- das Hebelelement (9) aufgrund der Gelenkeinrichtung (13) relativ zu dem Hebelträger (10) verschwenkbar ist.

5. Vorschaltkammer nach einem der vorstehenden Ansprüche, wobei
- das Hebelelement (9) eine längliche Erstreckung aufweist und an einem trägerseitigen Ende an dem Hebelträger (10) gehalten ist, und wobei
- an dem anderen, dem Hebelträger (10) gegenüberliegenden inhalatorseitigen Ende des Hebelelements (9) die Wirkfläche (11) ausgebildet ist.

6. Vorschaltkammer nach einem der vorstehenden Ansprüche, wobei eine Sicherungseinrichtung (15) vorgesehen ist, zum Abstützen des Inhalators (2) an wenigstens einer Seite, um ein Verdrehen des Inhalators (2) um eine Mittelachse der Gehäusekammer (5) relativ zu der Anschlusseinrichtung (7) zu verhindern.

7. Vorschaltkammer nach einem der vorstehenden Ansprüche, wobei an dem inhalatorseitigen Ende des Hebelelements (9) eine Abstützeinrichtung (16) vorgesehen ist, zum Abstützen des Inhalators (2) an seiner rückwärtigen, von der Lage des Hebelelements (9) abgewandten Seite.

8. Vorschaltkammer nach einem der vorstehenden Ansprüche, wobei die Sicherungseinrichtung (15) oder die Abstützeinrichtung (16) jeweils wenigstens eine Fläche aufweisen, die an dem inhalatorseitigen Ende des Hebelelements (9) ausgebildet ist.

9. Vorschaltkammer nach einem der vorstehenden Ansprüche, wobei die Sicherungseinrichtung eine Fläche (17) aufweist, die an der Anschlusseinrichtung (7) ausgebildet ist.

10. Vorschaltkammer nach einem der vorstehenden Ansprüche, wobei eine Verriegelungseinrichtung (19) vorgesehen ist, zum Verriegeln der Position des Hebelelements (9) in einer der beiden Stellungen.

11. Vorschaltkammer nach einem der vorstehenden Ansprüche, wobei die Verriegelungseinrichtung (19) zwischen zwei Zuständen bewegbar ist, nämlich einem Verriegelungszustand und einem Betriebszustand, wobei die Verriegelungseinrichtung in dem Verriegelungszustand zum Halten des Hebelelements (9) in einer vorgegebenen Stellung dient und in dem Betriebszustand eine Bewegung des Hebelelements freigibt.

12. Vorschaltkammer nach einem der vorstehenden Ansprüche, wobei
- die Hebeleinrichtung (9) einen Griffbereich (9a) aufweist, zum Greifen durch einen Bediener, und wobei
- die Lage der Wirkfläche (11) relativ zu dem Griffbereich (9a) veränderbar ist, zum Anpassen an unterschiedlich große Inhalatoren (2).

13. Vorschaltkammer nach einem der vorstehenden Ansprüche, wobei die Anschlusseinrichtung (7) an einer Stirnseite der Gehäusekammer (5) gehalten ist und an dieser Stirnseite in wenigstens drei Stellungen verdrehbar ist, nämlich in
- eine erste Drehstellung, in der der Inhalator (2) in der Anschlusseinrichtung (7) fest gehalten ist und die Anschlusseinrichtung (7) in der Gehäusekammer (5) fest gehalten ist,
- eine zweite Drehstellung, in der der Inhalator (2) aus der Anschlusseinrichtung (7) entfernbar ist und die Anschlusseinrichtung (7) in der Gehäusekammer (5) fest gehalten ist,
- eine dritte Drehstellung, in der die Anschlusseinrichtung (7) von der Stirnseite der Gehäusekammer (5) entfernbar ist.

14. Inhalationsvorrichtung, mit einer Vorschaltkammer (1) nach einem der vorstehenden Ansprüche und einem Inhalator (2), wobei
- der Inhalator (2) aufweist
+ einen ein Medikament aufnehmenden Behälter (3),
+ ein an dem Behälter (3) angeordnetes Mundelement und
+ eine wirkungsmäßig zwischen dem Behälter (3) und dem Mundelement angeordnete Dosiereinrichtung zum Erzeugen eines Aerosols, wobei das Aerosol eine vorbestimmte Menge des Medikaments enthält;
- der Inhalator (2) mit seinem Mundelement an der Anschlusseinrichtung (7) befestigbar ist, und wobei
- das Auslöseelement (3a) des Inhalators eine Bodenfläche des Behälters (3) ist.

## Claims

1. A spacer chamber (1) for an inhaler (2), comprising
- a housing chamber (5),
- a mouthpiece (6) arranged on the housing chamber (5),
- a connecting means (7) arranged on the housing chamber (5) to connect a mouth element provided on the inhaler (2), and comprising
- an operating means held on the housing chamber (5) which is movable to two positions and has an active surface (11) that is arranged in such a manner that the active surface (11) can act on an activation element (3a) of the inhaler (2) in any one of the positions of the operating means , if the inhaler (2) is connected to the connecting means (7),
wherein
- the operating means includes a lever means (8),
- the lever means (8) includes a lever element (9) and a lever carrier (10) carrying the lever element (9), and wherein
- the lever element (9) is movable, in particular swivelable, relative to the lever carrier (10), **characterized in that** the lever carrier (10) is fastened on the housing chamber (5) close to the mouthpiece (6).

2. The spacer chamber according to claim 1, wherein the connecting means (7)
- is shaped annular,
- is releasably fastenable to the housing chamber (5), and
- is formed to releasably carry the inhaler (2).

3. The spacer chamber according to any one of the preceding claims, wherein the lever carrier (10) is fastened, in particular, is fastened releasably, on the housing chamber (5).

4. The spacer chamber according to any one of the preceding claims, wherein
- a hinge means (13) is arranged between the lever element (9) and the lever carrier (10),
- the lever element (9) is swivelable relative to the lever carrier (10) due to the hinge means (13).

5. The spacer chamber according to any one of the preceding claims, wherein
- the lever element (9) has an elongated extension and is held on the level carrier (10) on a carrier-sided end, and wherein
- the active surface (11) is formed on the other inhaler-sided end of the lever element (9) opposite to the lever carrier (10).

6. The spacer chamber according to any one of the preceding claims, wherein a safety means (15) is provided -to support the inhaler (2) on at least one side- in order to prevent the inhaler (2) from twisting around a middle axis of the housing chamber (5) relative to the connecting means (7).

7. The spacer chamber according to any one of the preceding claims, wherein a supporting means (16) is provided on the inhaler-sided end of the lever element (9) to support the inhaler (2) at its rearward side facing away from the position of the lever element (9).

8. The spacer chamber according to any one of the preceding claims, wherein the safety means (15) or the supporting means (16) each have at least one surface formed on the inhaler-sided end of the lever element (9).

9. The spacer chamber according to any one of the preceding claims, wherein the safety means has a surface (17) formed on the connecting means (7).

10. The spacer chamber according to any one of the preceding claims, wherein a locking means (19) is provided to lock the position of the lever element (9) in one of the two positions.

11. The spacer chamber according to any one of the preceding claims, wherein the locking means (19) is movable between two states, namely a locking state and an operating state, wherein the locking means in the locking state serves to hold the lever element (9) in a predetermined position and releases a movement of the lever element in the operating state.

12. The spacer chamber according to any one of the preceding claims, wherein
- the lever means (9) includes a grip area (9a) to be gripped by an operator, and wherein
- the position of the active area (11) is changeable relative to the grip area (9a) to be adapted to inhalers (2) of different sizes.

13. The spacer chamber according to any one of the preceding claims, wherein the connecting means (7) is held on a front side of the housing chamber (5) and is twistable on this front side to at least three positions, namely in
- a first rotational position in which the inhaler (2) is held tightly within the connecting means (7), and the connecting means (7) is held tightly within the housing chamber (5),
- a second rotational position in which the inhaler (2) is removable from the connecting means (7), and the connecting means (7) is held tightly within the housing chamber (5),
- a third rotational position in which the connecting means (7) is removable from the front side of the housing chamber (5).

14. An inhalation device comprising a spacer chamber (1) according to any one of the preceding claims and an inhaler (2), wherein
- the inhaler (2) includes
+ a container (3) accommodating a medicinal product,
+ a mouth element arranged on the container (3), and
+ a dosage means functionally arranged between the container (3) and the mouth element to produce an aerosol, with the aerosol containing a predetermined amount of the medicinal product;
- the inhaler (2) with its mouth element is fastenable on the connecting means (7), and wherein
- the activation element (3a) of the inhaler is a bottom surface of the container (3).

## Revendications

1. Chambre d'inhalation (1) pour un inhalateur (2), avec
- une chambre de boîtier (5),
- un embout buccal (6) disposé sur la chambre de boîtier (5),
- un dispositif de raccordement (7), disposé sur la chambre de boîtier (5), pour raccorder un élément buccal prévu sur l'inhalateur (2), et avec
- un dispositif de commande, monté sur la chambre de boîtier (5), qui est apte à être amené dans deux positions et qui présente une surface active (11) disposée de manière à pouvoir agir sur un élément de déclenchement (3a) de l'inhalateur (2) dans l'une des positions du dispositif de commande, quand l'inhalateur (2) est raccordé au dispositif de raccordement (7),
- le dispositif de commande comportant un dispositif à levier (8),
- le dispositif à levier (8) comportant un élément de levier (9) et un support de levier (10) qui porte ledit élément de levier (9), et
- l'élément de levier (9) étant mobile, en particulier apte à pivoter, par rapport au support de levier (10), **caractérisée en ce que** le support de levier (10) est fixé à la chambre de boîtier (5) près de l'embout buccal (6).

2. Chambre d'inhalation selon la revendication 1, le dispositif de raccordement (7)
- ayant une forme annulaire,
- étant apte à être fixé de manière amovible à la chambre de boîtier (5), et
- étant conçu pour porter l'inhalateur (2) de manière amovible.

3. Chambre d'inhalation selon l'une des revendications précédentes, le support de levier (10) étant fixé, en particulier de manière amovible, à la chambre de boîtier (5)

4. Chambre d'inhalation selon l'une des revendications précédentes,
- un dispositif articulé (13) étant disposé entre l'élément de levier (9) et le support de levier (10),
- l'élément de levier (9) étant apte à pivoter grâce au dispositif articulé (13) par rapport au support de levier (10).

5. Chambre d'inhalation selon l'une des revendications précédentes,
- l'élément de levier (9) présentant une forme allongée et étant monté, à une extrémité située côté support, sur le support de levier (10), et
- la surface active (11) étant formée sur l'autre extrémité de l'élément de levier (9), opposée au support de levier (10) et située côté inhalateur.

6. Chambre d'inhalation selon l'une des revendications précédentes, un dispositif d'arrêt (15) étant prévu pour l'appui de l'inhalateur (2) sur au moins un côté, afin d'empêcher ledit inhalateur (2) de tourner sur un axe médian, par rapport au dispositif de raccordement (7).

7. Chambre d'inhalation selon l'une des revendications précédentes, un dispositif d'appui (16) étant prévu sur l'extrémité de l'élément de levier (9) située côté inhalateur, pour l'appui de l'inhalateur (2) sur un côté arrière opposé à la position de l'élément de levier (9).

8. Chambre d'inhalation selon l'une des revendications précédentes, le dispositif d'arrêt (15) ou le dispositif d'appui (16) présentant au moins une surface qui est formée sur l'extrémité de l'élément de levier (9) située côté inhalateur.

9. Chambre d'inhalation selon l'une des revendications précédentes, le dispositif d'arrêt présentant une surface (17) qui est formée sur le dispositif de raccordement (7).

10. Chambre d'inhalation selon l'une des revendications précédentes, un dispositif de verrouillage (19) étant prévu pour verrouiller la position de l'élément de levier (9) dans l'une des deux positions.

11. Chambre d'inhalation selon l'une des revendications précédentes, le dispositif de verrouillage (19) étant mobile entre deux états : un état de verrouillage et un état de fonctionnement, le dispositif de verrouillage servant, dans l'état de verrouillage, à retenir l'élément de levier (9) dans une position prédéfinie, et autorisant, dans l'état de fonctionnement, un mouvement dudit élément de levier.

12. Chambre d'inhalation selon l'une des revendications précédentes,
- le dispositif de levier (9) comportant une zone de préhension (9a) à saisir par un utilisateur, et
- la position de la surface active (11) étant apte à être modifiée par rapport à ladite zone de préhension (9a), pour s'adapter à des inhalateurs (2) de tailles différentes.

13. Chambre d'inhalation selon l'une des revendications précédentes, le dispositif de raccordement (7) étant monté sur un côté frontal de la chambre de boîtier (5) et étant apte à tourner, sur ce côté frontal, jusqu'à au moins trois positions :
- une première position de rotation dans laquelle l'inhalateur (2) est retenu dans le dispositif de raccordement (7) et ce dernier est retenu dans la chambre de boîtier (5),
- une deuxième position de rotation dans laquelle l'inhalateur (2) est apte à être enlevé du dispositif de raccordement (7) et ce dernier (7) est retenu dans la chambre de boîtier (5),
- une troisième position de rotation dans laquelle le dispositif de raccordement (7) est apte à être enlevé du côté frontal de la chambre de boîtier (5).

14. Dispositif d'inhalation avec une chambre d'inhalation (1) selon l'une des revendications précédentes, et avec un inhalateur (2),
- l'inhalateur (2) comportant
+ un récipient (3) contenant un médicament,
+ un élément buccal disposé sur le récipient (3), et
+ un dispositif de dosage disposé, en termes d'action, entre le récipient (3) et l'élément buccal, pour produire un aérosol, l'aérosol contenant une quantité prédéfinie de médicament ;
- l'inhalateur (2) étant apte à être fixé avec son élément buccal au dispositif de raccordement (7), et
- l'élément de déclenchement (3a) de l'inhalateur étant une surface de fond du récipient (3).
